# EUROPEAN PATENT APPLICATION

(11) **EP 4 454 715 A1**
(43) Date of publication of application: **30.10.2024**
(21) Application number: 22910758.6
(22) Date of filing: 28.11.2022
(51) Int. Cl.: A61Q 13/00, C11D 3/08, C11D 7/14, B01J 13/14, C11B 9/00

(54) **COMPOSITION**

(30) Priority: 23.12.2021 JP 2021209133
(71) Applicant: Kao Corporation, Tokyo 103-8210 (JP)
(72) Inventor: SUZUKI Masahiro, Wakayama-shi, Wakayama 640-8580 (JP); TAKUMI Hiroki, Wakayama-shi, Wakayama 640-8580 (JP)
(74) Representative: Hoffmann Eitle
(86) International application number: PCT/JP2022/043646
(87) International publication number: WO 2023/120040

(57) **Abstract**

The present invention is a composition containing (A) a silica capsule enclosing a functional agent, (B) a compound selected from a silicic acid and a silicate, and water, wherein component (B) is contained in the composition separately from component (A).

## Description

### Field of the Invention

The present invention relates to a composition containing a capsule enclosing a functional agent.

### Background of the Invention

Products for various applications are formulated with functional agents such as fragrances, sensory agents, moisturizers, bactericides and others. For example, fragrances are used in products such as fabric softeners, laundry detergents, body detergents and others for the purpose of giving scents to the products themselves, clothing, the body and others. In that case, the ability to stably retain the fragrances is required to prevent the scents from disappearing in the products. In order to maintain effects of such functional agents, attempts have been made to encapsulate functional agents in microcapsules and incorporate them into products.

Further, conventionally used microcapsules whose wall materials are resins such as melamine and others may fall under the category of microplastics if rules are revised in light of growing social environmental awareness in the future or the like, and there are concerns about their environmental impact. On the other hand, silica capsules whose wall materials are inorganic compounds do not fall under the category of microplastics, and reduced environmental impact can be expected if those capsules can be incorporated into products.

JP-A 2015-128762 discloses microcapsules obtained by a predetermined production process, the microcapsules each containing, a core containing at least one organic compound, a first shell encapsulating the core and containing silica as a constitutional component thereof, and a second shell encapsulating the first shell and containing silica as a constitutional component thereof, wherein the microcapsules have an average particle size of not less than 0.5 um and not more than 50 µm.

JP-A 2009-504812 discloses an aqueous liquid washing and cleaning agent containing surfactants as well as further usual ingredients of washing and cleaning agents, the agent containing at least one capsule, the capsule containing an active ingredient, an aluminum silicate and a silicic acid in a matrix, the aluminum silicate and the silicic acid being present in a ratio from 1:10 to 10:1.

JP-A 2011-517323 discloses a fragrance carrier system containing an encapsulated fragrance composition, wherein the fragrance composition contains an emulsion of a fragrance compound in an aqueous medium and is encapsulated within a shell containing a silicon-containing material, the shell having a mean diameter size which is lower than 30 micrometers, and a surfactant composition containing the fragrance carrier system.

### Summary of the Invention

The present invention provides a composition which contains a silica capsule enclosing a functional agent and water and is more excellent in storage stability at high temperatures.

The present invention relates to a composition containing the following components (A) and (B) and water,
component (A): a silica capsule enclosing a functional agent, and
component (B): a compound selected from a silicic acid and a silicate,
wherein component (B) is contained in the composition separately from component (A).

According to the present invention, provided is a composition which contains a silica capsule enclosing a functional agent and water and is more excellent in storage stability at high temperatures.

### Embodiments of the Invention

Examples of the silica capsule enclosing a functional agent of component (A) include, for example, a capsule having a shell containing silica as a constituent component and a core containing the functional agent inside the shell.

### (Shell)

Examples of component (A) include component (A) having a shell containing silica as a constituent component. The shell of component (A) may be a shell in which part or substantially all of constituent structures are made by using silica as a constituent component. From the viewpoint of high-temperature storage stability, silica is preferably produced from a raw material silica such as an alkoxysilane or the like which produces a silanol compound on hydrolysis. From the viewpoint of high-temperature storage stability, the shell of component (A) of the present invention is preferably formed by a sol-gel reaction using an alkoxysilane as a precursor. In the present invention, the "sol-gel reaction" means a reaction in which an alkoxysilane goes through a sol or gel state on hydrolysis and polycondensation reactions to form silica of a constituent component of the shell. Specifically, for example, a tetraalkoxysilane is hydrolyzed, a silanol compound produces a siloxane oligomer through a dehydration condensation reaction and an alcohol-elimination condensation reaction, and the dehydration condensation reaction further proceeds to form silica.

Examples of the raw material silica include, for example, at least one selected from the group composed of silicon tetrachloride, tetraalkoxysilanes, alkyl alkoxysilanes, water glass and metal silicates. Among these, a tetraalkoxysilane or an alkyl alkoxysilane is preferable and a tetraalkoxysilane is more preferable from the viewpoint of high-temperature storage stability.

Specific examples of the tetraalkoxysilanes include tetramethoxysilane, tetraethoxysilane, tetrapropoxysilane and others. Tetramethoxysilane or tetraethoxysilane is preferable and tetraethoxysilane is more preferable from the viewpoint of high-temperature storage stability.

Specific examples of the alkyl alkoxysilanes include methyltrimethoxysilane, methyltriethoxysilane, ethyltrimethoxysilane, ethyltriethoxysilane, phenyltrimethoxysilane, phenyltriethoxysilane, dimethyldimethoxysilane, methylphenyldimethoxysilane, dimethyldiethoxysilane, diphenyldiethoxysilane, methylphenyldiethoxysilane, ethylphenyldimethoxysilane, diethyldiethoxysilane, ethylphenyldiethoxysilane, trimethylmethoxysilane, trimethylethoxysilane, dimethylphenylethoxysilane, triethylmethoxysilane, triethylethoxysilane and others.

One of them can be used alone or a combination of two or more of them can be used. Further, a condensate of them or the like can also be used.

Further, the shell of the silica capsule of the present invention may contain an inorganic polymer other than silica as a constituent component in the range that the effects of the present invention are not impaired. In the present invention, the inorganic polymer refers to a polymer containing an inorganic element. Examples of the inorganic polymer include a polymer composed of an inorganic element alone, a polymer whose main chain is composed of an inorganic element alone and which has an organic group as a side chain or a substituent, or the like.

From the viewpoint of high-temperature storage stability, the inorganic polymer is preferably a metal oxide containing a metal element or a semimetal element, and further preferably a polymer formed by a reaction similar to the aforementioned sol-gel reaction for silica using a metal alkoxide [M(OR)ₓ] as a precursor. Here, M is a metal or semimetal element, and R is a hydrocarbon group.

Examples of the metal or semimetal element constituting the metal alkoxide include titanium, zirconium, aluminum, zinc or the like.

The shell may have a first shell and a second shell, and component (A) may have the first shell enveloping the core containing one or more functional agents and the second shell enveloping the first shell. Further, component (A) of the present invention may have a third shell composed of an organic polymeric compound in contact with the second shell. Such a multi-layer shell can retain functional agents such as fragrances or the like for a long period, and is preferable from the viewpoint of high-temperature storage stability.

A thickness of the shell (which is the first shell when the shell has the first and second shells) is preferably 5 nm or more, and preferably 20 nm or less and more preferably 15 nm or less from the viewpoint of high-temperature storage stability. Further, from the viewpoint of high-temperature storage stability, the shell (which is the first shell when the shell has the first and second shells) is preferably a dense layer having as less pores as possible for long-time retention of the enclosed functional agent.

When component (A) has the second shell, a thickness of the second shell is preferably 10 nm or more and more preferably 20 nm or more, and preferably 100 nm or less and more preferably 80 nm or less from the viewpoint of high-temperature storage stability. From the viewpoint of high-temperature storage stability, the second shell is preferably of a mesoporous structure, which is a higher order structure in which silica is present not only in a direction along an interface with the first shell but also in a thickness direction.

Here, the "mesoporous structure" in the second shell refers to a structure that a diameter of pores present within the structure (so-called mesopores) falls within the range of preferably more than 2 nm, more preferably 10 nm or more and further preferably 30 nm or more, and preferably 50 nm or less, more preferably 45 nm or less and further preferably 40 nm or less from the viewpoint of high-temperature storage stability.

Component (A) has high mechanical strength as the second shell is of the mesoporous structure.

Average thicknesses of the first and second shells of component (A) and pore diameters of the first and second shells can be measured by observation with a transmission electron microscope (TEM). Specifically, thicknesses of the first shell and the second shell and pore diameters of the first shell and the second shell are actually measured on a photograph under observation with a transmission electron microscope. This operation is performed by changing a field of view five times. Distributions of the thicknesses and the pore diameters of the first shell and the second shell are determined from the obtained data. A magnification of the transmission electron microscope is 10,000 to 100,000 times as a guide, but is appropriately adjusted depending on a size of component (A). Here, as the transmission electron microscope (TEM), for example, the trade name "JEM-2100" (manufactured by JEOL Ltd.) can be used.

### (Core)

The core of component (A) of the present invention contains one or more functional agents. The functional agents may be, for example, oil-soluble liquids. When a fragrance is used as such a functional agent, component (A) encloses the fragrance inside the shell, so that when the shell is broken, the fragrance is released and gives off a scent.

Examples of the functional agents include, for example, one or more selected from the group composed of a fragrance, a fragrance precursor, an oil agent, an antioxidant, a cooling sensation agent, a warming sensation agent, an antibacterial agent, a dye, a colorant, an ultraviolet absorber, a silicone, a solvent and an oil-soluble polymer, further one or more selected from the group composed of a fragrance, a fragrance precursor, an oil agent, an antioxidant, a cooling sensation agent, a warming sensation agent, an antibacterial agent, an ultraviolet absorber and a solvent, and further one or more selected the group composed of a fragrance and a fragrance precursor. Further, one such functional agent may be a skincare component such as a moisturizer or the like, a cosmetic oil, an antiseptic, an antioxidant, an insecticidal component or an insect-repellant component.

Examples of the fragrance include, for example, γ-undecalactone, 2-cyclohexylidene-2-phenylacetonitrile, damascenones, δ-damascone, α-methyl-β-(p-t-butylphenyl)-propionaldehyde, β-ionone, myrrh aldehyde, ethyl tricyclo[5.2.1.0-2,6]decane-2-carboxylate, citronellol, geraniol, α-ionone, patchouli alcohol, 6,7-dihydro-1,1,2,3,3-pentamethyl-4(5H)-indanone, methyl dihydrojasmonate, hexyl cinnamic aldehyde, amyl cinnamic aldehyde, allyl cyclohexyl propionate, dimethylbenzyl carbinyl butyrate, tricyclodecenyl propionate, amyl salicylate, γ-methylionone, α-damascone, β-damascone, Nerolin Yara Yara, phenylhexanol, 2-methyl-4-(2,2,3-trimethyl-3-cyclopenten-1-yl)-2-buten-1-ol, dodecahydro-3a,6,6,9a-tetramethylnaphtho[2,1-b]furan, γ-nonalactone, methyl β-naphthyl ketone, eugenol, Lyral, dimethyl benzyl carbinyl acetate, iso-damascone, 2-cyclohexylidene-2-phenylacetonitrile, γ-decalactone, α-methyl-3,4-methylenedioxyhydrocinnamicaldehyde, 7-methyl-3,5-dihydro-2H-benzodioxepinon, tricyclodecenyl acetate (tricyclodecenyl acetate), tricyclodecenyl propionate, allyl 2-pentyloxy glycolate, 1-(2-tert-butylcyclohexyloxy)-2-butanol, citronellyloxyacetaldehyde, indole, 4-methyl-3-decen-5-ol, para-menthane-8-thiol-3-one, 3-(para-tert-butylphenyl)-propanal, ethyl cinnamate, 5-methyl-3-heptanone oxime, methyl anthranilate, terpineol, β-caryophyllene, citronellyl acetate, geranyl acetate, neryl acetate, p,t-butylcyclohexyl acetate, o,t-butylcyclohexyl acetate, tetrahydrogeraniol, 2-isobutyl-4 hydroxy-4-methyltetrahydropyranol (Florosa), α-dynascone, cis-jasmone, bicyclo[3.2.1]octan-8-one-1,5-dimethyl-oxime, 2,4-dimethyl-4,4α,5,9β-tetrahydroindeno[1,2-d]-m-dioxin, 3-(para-ethylphenyl)-2,2-dimethylpropanal, ethyl-2-tert-butylcyclohexyl-carbonate, hexyl benzoate, 4-acetoxy-3-amyltetrahydropyran, dodecyl aldehyde, dihydro-β-ionone, methyl cyclooctyl carbonate, ethyl methylphenylglycidate, isoeugenol, diphenyl oxide, 2,2,5-trimethyl-5-pentylcyclopentanone, thymol, nerolin bromelia, 5,6-dimethyl-8-isopropenyl, bicyclo[4,4,0]-1-decen-3-one, 3-(4-isopropylphenyl)-propanal, 4-isopropylcyclohexanemethanol, methyl methyl anthranilate, dodecanenitrile 3-dodecenal, octanal, nonanal, decanal, lilial, p,t-butylhydrocinnamic aldehyde, dimethyl tetrahydrobenzaldehyde, hexyl acetate, linalyl acetate, terpinyl acetate, allyl caproate, hexyl salicylate, benzyl salicylate, cyclohexyl salicylate, cis-3-hexenyl salicylate, methyl dihydrojasmonate, cyclamen aldehyde, limonene, linalool, tetrahydrolinalool, dihydromyrcenol, methyl β-naphthyl ketone, Iso E Super, cedryl methyl ether, Javanol (manufactured by Givaudan), ambroxane, 1,8-cineole, geranyl nitrile, citronellyl nitrile, 11-oxa-16-hexadecanolide (Musk R1, manufactured by Givaudan), ethylene brassylate, ethylene dodecanedioate, cashmeran, cyclopentadecanolide, cyclohexadecanolide, ambrettolide or the like. The fragrance may be a fragrance composition containing multiple fragrances.

Examples of the fragrance precursor include, for example, a compound which releases a fragrance component in response to water, or the like. Specific examples include a silicic acid ester compound having an alkoxy component derived from a fragrance alcohol, a fatty acid ester compound having an alkoxy component derived from a fragrance alcohol, an acetal compound or a hemiacetal compound obtained by a reaction of a carbonyl component derived from a fragrance aldehyde or a fragrance ketone with an alcohol compound, a Schiff base compound obtained by a reaction of a carbonyl component derived from a fragrance aldehyde or a fragrance ketone with a primary amine compound, or a hemiaminal compound or a hydrazone compound obtained by a reaction of a carbonyl component derived from a fragrance aldehyde or a fragrance ketone with a hydrazine compound.

Further, examples of another form of the fragrance precursor include a compound which releases a fragrance component in response to light. Examples include, for example, a 2-nitrobenzyl ether compound having an alkoxy component derived from a fragrance alcohol, an α-ketoester compound having a carbonyl component derived from a fragrance aldehyde or a fragrance ketone, or a coumaric acid ester compound having an alkoxy component derived from a fragrance alcohol. These fragrance precursors may be used as polymers such as, for example, reaction products obtained by reactions of some carboxy groups of polyacrylic acid with fragrance alcohols, and others. Among these, a silicic acid ester compound having an alkoxy component derived from a fragrance alcohol is preferable.

The functional agents have a ClogP value of preferably 2 or more, more preferably 3 or more and further preferably 4 or more, and preferably 30 or less, more preferably 20 or less and further preferably 10 or less. When the functional agents have a ClogP value of 2 or more, an encapsulation rate (hereinafter also referred to as an "enclosure rate") of the functional agents into component (A) is improved. Here, the same applies to the case where the functional agents are fragrance compositions containing multiple fragrances, and when the fragrance compositions have a ClogP value of 2 or more, an encapsulation rate (enclosure rate) of the fragrance compositions into component (A) can be improved.

Here, the ClogP value is a "calculated logP (ClogP)" calculated by the method described in A. Leo, "Comprehensive Medicinal Chemistry," Vol.4, (C. Hansch, P. G. Sammes, J. B. Taylor and C. A. Ramsden, Eds.), p. 295, Pergamon Press, 1990, and is a ClogP value calculated by the program CLOGP v4.01. In the case of a fragrance composition containing multiple fragrances, a CLogP value of the fragrance composition can be determined as a sum of CLogP values of the fragrances multiplied by their respective volume ratios in the fragrance composition.

Further, the functional agents have an oil-water interfacial tension of preferably 7 mN/m or more, more preferably 10 mN/m or more and further preferably 13 mN/m or more at 25°C from the viewpoint of retention of the functional agents. The oil-water interfacial tension of the functional agents can be measured, for example, by the contact angle meter "DropMaster DM-501" (trade name, manufactured by Kyowa Interface Science Co., Ltd.)

A volume average particle size of component (A) is preferably 0.5 um or more, more preferably 0.7 um or more and further preferably 1 um or more, and preferably 50 um or less, more preferably 10 um or less and further preferably 5 um or less from the viewpoint of incorporation into products and the viewpoint of retention of the functional agents.

Note that, in the present invention, the volume average particle size of component (A) can be measured by the method described in Examples. For example, it can be measured using the laser diffraction/scattering particle size distribution measurement device "LA-960" (trade name, manufactured by HORIBA, Ltd.) In that case, for measurements, a flow cell is used, the medium is water, and the refractive index is set at 1.40-0i. A dispersion liquid containing component (A) is added to the flow cell, measurements are made at a concentration at which a transmittance near 90% is indicated, and an average particle size is determined on a volume basis.

When component (A) is component (A) having the first shell and the second shell containing silica as a constituent component and the core containing one or more functional agents inside the first shell, component (A) can be obtained, for example, by a production method including the following steps (1) and (2),
step (1): a step of forming a capsule enclosing the one or more functional agents, in which an organic phase containing the functional agents and a raw material silica (for example, a tetraalkoxysilane) is mixed and emulsified in an aqueous phase containing a surfactant (for example, a cationic surfactant), and a sol-gel reaction is thereafter carried out under an acidic condition to form a shell, and
step (2): a step of forming a capsule having the second shell enveloping the first shell, in which a raw material silica (for example, a tetraalkoxysilane) is further added to a dispersion liquid containing the capsule obtained in step (1), and a sol-gel reaction is carried out.

More specifically, component (A) can be obtained, for example, by a production method including the following steps (1a) and (2a),
step (1a): a step of forming a capsule having the core and the first shell, in which an organic phase containing the one or more functional agents and a tetraalkoxysilane in an amount of 10 mass% or more and 60 mass% or less relative to the functional agents is emulsified in an aqueous phase containing a surfactant (for example, a cationic surfactant), and a sol-gel reaction is carried out under an acidic condition, and
step (2a): a step of forming a capsule having the second shell enveloping the first shell, in which a tetraalkoxysilane is further added to an aqueous dispersion containing the capsule obtained in step (1a), and a sol-gel reaction is carried out in such a manner that an initial pH of the sol-gel reaction of step (2a) is maintained lower than an initial pH of the sol-gel reaction of step (1a),
the production method further including the following step (3a) as necessary,
step (3a): a step of forming a capsule having the third shell, in which a dispersion liquid containing the capsule obtained in step (2a) is mixed with an aqueous solution of an organic polymeric compound (for example, an anionic synthetic polymeric compound).

Here, the "sol-gel reactions" in steps (1) and (2) and steps (1a) and (2a) are reactions of synthesizing silica of the first and second shells, in which a raw material silica (silica precursor) is polymerized with alcohol elimination through hydrolysis and polycondensation under an acidic condition.

The above production methods can be performed with reference to, for example, JP-A 2015-128762, JP-A 2017-114802 or the like. In the above production methods, component (A) is usually obtained in a state of being dispersed in water. This aqueous dispersion can be used as-is depending on applications, but in some cases, component (A) is separated and used. As a separation method, filtration, centrifugation or the like can be employed.

A content of the functional agents in component (A) may be, for example, 5 mass% or more, further 10 mass% or more and further 12 mass% or more, and 50 mass% or less, further 45 mass% or less and further 40 mass% or less.

In the composition of the present invention, a content of component (A) in terms of the enclosed functional agents can be preferably 0.02 mass% or more, more preferably 0.05 mass% or more, further preferably 0.1 mass% or more and furthermore preferably 0.2 mass% or more, and preferably 10 mass% or less, more preferably 5 mass% or less and further preferably 1 mass% or less in the composition from the viewpoint of high-temperature storage stability.

Component (B) is a compound selected from a silicic acid and a silicate. Examples of component (B) include a compound represented by the following formula (B1),

M₂O·nSiO₂·mH₂O (B1)

wherein M is an atom selected from an alkali metal atom and a hydrogen atom, n is a number of 1.0 or more and 4.0 or less, and m is a number of 5.0 or more and 50.0 or less.

In the formula (B1), examples of the alkali metal atom of M include a sodium atom or a potassium atom.

In the formula (B1), n is a number of preferably 1.8 or more and 4.0 or less, more preferably from 1.9 to 3.5 and further preferably 2.0 or more and 3.3 or less from the viewpoint of high-temperature storage stability. m is a number of preferably 10.0 or more and 48.0 or less, more preferably from 11.0 to 35.0 and further preferably 11.5 or more and 30.0 or less.

Component (B) is preferably sodium silicate (sodium silicate) from the viewpoint of high-temperature storage stability. As sodium silicate, those which are commercially available, for example, as sodium silicate No. 1, sodium silicate No. 2, sodium silicate No. 3, sodium silicate No. 4, sodium silicate No. 5 and others can be used. Further, as sodium silicate, those which are specified in JIS K 1408 can be used.

Specific examples of component (B) include the compounds shown in the following table:

| Example of silicate | | Composition (mass%) | | | | | Molar ratio | |
|---|---|---|---|---|---|---|---|---|
| | | Na₂O | SiO₂ | K₂O | H₂O | Total | n(SiO₂/M₂O) | m(H₂O/M₂O) |
| Sodium silicate | Example 1 | 15.6 | 31.8 | | 52.6 | 100.0 | 2.1 | 11.6 |
| | Example 2 | 11.8 | 28.6 | | 59.6 | 100.0 | 2.5 | 17.3 |
| | Example 3 | 9.5 | 29.1 | | 61.4 | 100.0 | 3.2 | 22.3 |
| | Example 4 | 7.7 | 25.0 | | 67.4 | 100.0 | 3.4 | 30.2 |
| | Example 5 | 7.1 | 25.7 | | 67.2 | 100.0 | 3.8 | 32.7 |
| Potassium silicate | Example 6 | | 28.0 | 22.0 | 50.0 | 100.0 | 2.0 | 11.9 |
| | Example 7 | | 21.0 | 9.0 | 70.0 | 100.0 | 3.7 | 40.7 |
| Sodium metasilicate | Example 8 | 28.9 | 27.5 | | 43.6 | 100.0 | 1.0 | 5.2 |
| | Example 9 | 22.0 | 21.0 | | 57.0 | 100.0 | 1.0 | 8.9 |

The composition of the present invention can contain component (B) in an amount of preferably 0.001 mass% or more, more preferably 0.01 mass% or more, further preferably 0.05 mass% or more and furthermore preferably 0.15 mass% or more, and preferably 0.5 mass% or less, more preferably 0.4 mass% or less and further preferably 0.3 mass% or less in the composition from the viewpoint of high-temperature storage stability. Note that a content of component (B) in the composition in the present invention is a content in terms of silicon dioxide (SiO₂), and specifically quantified by the method described later in Examples.

In the composition of the present invention, a mass ratio of a content of component (B) in terms of silicon dioxide (SiO₂) to a content of component (A) in terms of the enclosed functional agents, (B)/(A), is preferably 0.01 or more, more preferably 0.05 or more, further preferably 0.25 or more and furthermore preferably 0.75 or more, and preferably 1.0 or less from the viewpoint of high-temperature storage stability.

Component (B) is contained in the composition separately from component (A), in other words, exists in the composition without being enclosed in the silica capsule of component (A). The above percentage or mass ratio for component (B) is based on an amount of component (B) contained in the composition separately from component (A).

The composition of the present invention can further contain the following component (C) from the viewpoint of high-temperature storage stability, provided that surfactants enclosed in component (A) are excluded from component (C),
component (C): a surfactant.

From the viewpoints of dispersion stability of component (A) or other base agents in the composition, detergency when the composition is used as a detergent, and high-temperature storage stability, component (C) is preferably one or more surfactants selected from (C1) an anionic surfactant (hereinafter referred to as component (C1)) and (C2) a nonionic surfactant, and more preferably one or more surfactants selected from (C2) a nonionic surfactant (hereinafter referred to as component (C2)).

From the viewpoint of high-temperature storage stability, examples of the anionic surfactant of component (C1) include a sulfonic acid having a hydrocarbon group and a salt thereof, a sulfate having a hydrocarbon group and a salt thereof, or a carboxylic acid having a hydrocarbon group and a salt thereof, and a sulfonate having a hydrocarbon group or a carboxylate having a hydrocarbon group is preferable. The hydrocarbon groups may be alkyl groups or alkenyl groups. The hydrocarbon groups have, for example, 3 or more, preferably 7 or more, more preferably 9 or more and further preferably 11 or more, and preferably 22 or less, more preferably 20 or less and further preferably 18 or less carbons from the viewpoint of high-temperature storage stability. Examples of the salts include monovalent metal salts such as sodium salts, potassium salts or the like, divalent metal salts such as magnesium salts or the like, ammonium salts, or organic amine salts such as monoethanolamine salts, diethanolamine salts, triethanolamine salts or the like, and from the viewpoint of high-temperature storage stability, sodium salts are preferable.

From the viewpoint of high-temperature storage stability, component (C1) is preferably one or more anionic surfactants selected from the following components (c1-1) to (c1-5), and more preferably one or more anionic surfactants selected from component (c1-1),
component (c1-1): a sulfonic acid or a salt thereof represented by the following general formula (c1-1),

   R¹-B-SO₃M (c1-1)
wherein in the formula (c1-1), R¹ represents an alkyl group or an alkenyl group with 3 or more and 21 or less carbons, B represents a benzene ring, M represents a hydrogen atom, an alkali metal, an alkaline earth metal (1/2 atom), ammonium or an organic ammonium, and the sulfonic acid group is bonded in an ortho, meta or para position relative to R¹ bonded to B,
component (c1-2): a salt of an internal olefin sulfonic acid with 14 or more and 24 or less carbons,
component (c1-3): a salt of a fatty acid with 8 or more and 20 or less carbons,
component (c1-4): a sulfate or a salt thereof represented by the following general formula (c1-4),

   R²-O-[(PO)ₘ/(EO)ₙ]-SO₃M (c1-4)
wherein in the formula (c1-4), R² represents an alkyl group or an alkenyl group with 8 or more and 22 or less carbons, in which a carbon atom bonded to the oxygen atom is a primary carbon atom, PO represents propyleneoxy group, EO represents ethyleneoxy group, EO and PO may be bonded in blocks or at random, / is a sign indicating that PO and EO are bonded in any order, m and n are average numbers of added moles, where m is 0 or more and 5 or less and n is 0 or more and 16 or less, and M represents a hydrogen atom, an alkali metal, an alkaline earth metal (1/2 atom), ammonium or an organic ammonium, and
component (c1-5): an α-sulfo fatty acid ester or a salt thereof represented by the following general formula (c1-5),

   R³-CH(SO₃M)COOR⁴ (c1-5)
wherein in the formula (c1-5), R³ represents an alkyl group or an alkenyl group with 6 or more and 20 or less carbons, R⁴ represents an alkyl group with 1 or more and 6 or less carbons, and M represents a hydrogen atom, an alkali metal, an alkaline earth metal (1/2 atom), ammonium or an organic ammonium.

In the formula (c1-1), R¹ has 3 or more, preferably 5 or more, more preferably 6 or more and further preferably 7 or more, and 21 or less, preferably 20 or less, more preferably 19 or less and further preferably 18 or less carbons from the viewpoint of high-temperature storage stability.

In the formula (c1-1), M is preferably an alkali metal or an organic ammonium, and more preferably sodium from the viewpoint of high-temperature storage stability. Note that a content of component (c1-1) in the present invention is based on an amount of a compound in terms of a sodium salt.

Specific examples of component (c1-1) include an alkyl (with 4 or more carbons) benzene sulfonic acid or a cumene sulfonic acid.

The internal olefin sulfonate of component (c1-2) has 14 or more, preferably 16 or more and more preferably 18 or more, and 24 or less, more preferably 22 or less and further preferably 20 or less carbons from the viewpoint of high-temperature storage stability.

Examples of component (c1-2) also include, in addition to an internal olefin sulfonate, a hydroxy alkane sulfonate or an olefin sulfonate produced during synthesis.

Examples of the salt of component (c1-2) include an alkali metal salt such as sodium, potassium or the like, an alkaline earth metal salt such as calcium, magnesium or the like, an ammonium salt, or an organic ammonium salt, for example, an alkanol ammonium salt such as monoethanolammonium, diethanolammonium, triethanolammonium or the like. Examples preferably include an alkali metal salt, or an alkanol ammonium salt with 2 or more and 6 or less carbons from the viewpoint of high-temperature storage stability. Note that a content of component (c1-2) in the present invention is based on an amount of a compound in terms of a potassium salt.

The fatty acid of component (c1-3) has 8 or more, preferably 10 or more and more preferably 12 or more, and 20 or less, more preferably 18 or less and further preferably 16 or less carbons from the viewpoint of high-temperature storage stability.

Examples of the salt of component (c1-3) include an alkali metal salt, an alkaline earth metal (1/2 atom) salt, an ammonium salt or an organic ammonium salt from the viewpoint of high-temperature storage stability. Note that a content of component (c1-3) in the present invention is based on an amount of a compound in terms of acid form (hydrogen atom).

Specific examples of component (c1-3) include an octanoic acid salt, a decanoic acid salt, a lauric acid salt, a myristic acid salt, a palmitic acid salt, a stearic acid salt, a coconut fatty acid salt, a palm fatty acid salt, a palm kernel fatty acid salt or the like.

In the formula (c1-4), R² is an alkyl group or an alkenyl group with preferably 9 or more, more preferably 10 or more and further preferably 12 or more, and preferably 18 or less, more preferably 16 or less and further preferably 14 or less carbons from the viewpoint of high-temperature storage stability. R² is preferably an alkyl group, and from the viewpoint of high-temperature storage stability, R² is more preferably a linear alkyl group.

In the formula (c1-4), m is preferably 4 or less and more preferably 3 or less from the viewpoint of high-temperature storage stability.

In the formula (c1-4), n is preferably 0 or more, more preferably 1 or more, further preferably 2 or more and furthermore preferably 4 or more, and preferably 10 or less, more preferably 8 or less and further preferably 6 or less from the viewpoint of high-temperature storage stability.

In the formula (c1-4), M is preferably a hydrogen atom, an alkali metal such as sodium, potassium or the like, an alkaline earth metal (1/2 atom) such as magnesium, calcium or the like, or an organic ammonium from the viewpoint of high-temperature storage stability. M is more preferably an alkali metal such as sodium, potassium or the like, or an alkanol ammonium such as monoethanolammonium, diethanolammonium or the like, and further preferably sodium from the viewpoint of high-temperature storage stability.

Note that a content of component (c1-4) in the present invention is based on an amount of a compound in terms of a sodium salt.

From the viewpoint of high-temperature storage stability, specific component (c1-4) is preferably a (polyoxypropylene) polyoxyethylene alkyl ether sulfate sodium salt in which the alkyl group has 12 or more and 14 or less carbons, an average number of added moles of propyleneoxy group is 0 or more and 4 or less, and an average number of added moles of ethyleneoxy group is 1 or more and 4 or less. In other words, component (c1-4) is preferably a compound of the general formula (c1-4) in which R² is an alkyl group with 12 or more and 14 or less carbons, m is 0 or more and 4 or less, n is 1 or more and 4 or less, and M is sodium.

In the formula (c1-5), R³ is an alkyl group or an alkenyl group with preferably 8 or more and more preferably 10 or more, and preferably 18 or less and more preferably 16 or less carbons from the viewpoint of high-temperature storage stability. R³ is preferably an alkyl group.

In the formula (c1-5), R⁴ is an alkyl group with 1 or more, and preferably 5 or less and more preferably 4 or less carbons from the viewpoint of high-temperature storage stability.

In the formula (c1-5), M is preferably a hydrogen atom, an alkali metal such as sodium, potassium or the like, an alkaline earth metal (1/2 atom) such as magnesium, calcium or the like, or an organic ammonium from the viewpoint of high-temperature storage stability. M is more preferably an alkali metal such as sodium, potassium or the like, or an alkanol ammonium such as monoethanolammonium, diethanolammonium or the like, and further preferably sodium from the viewpoint of high-temperature storage stability.

Note that a content of component (c1-5) in the present invention is based on an amount of a compound in terms of a sodium salt.

From the viewpoint of high-temperature storage stability, specific component (c1-5) is preferably a sodium α-sulfo fatty acid methyl ester or a salt thereof of the formula (c1-5) in which R³ is an alkyl group with 11 or more and 14 or less carbons and R⁴ is a methyl group.

From the viewpoint of high-temperature storage stability, examples of the nonionic surfactant of component (C2) include a sucrose fatty acid ester, a glycerin fatty acid ester, a sorbitan fatty acid ester, a polyoxyalkylene sorbitan fatty acid ester, a polyoxyalkylene fatty acid ester, a fatty acid alkanolamide or an alkylene oxide adduct thereof, a polyoxyalkylene alkyl ether, an alkyl glycoside, a polyoxyalkylene alkyl ether, a glyceryl monoether or the like, and a polyoxyalkylene ether or a fatty acid methyl ester alkoxylate is preferable.

From the viewpoint of high-temperature storage stability, examples of component (C2) include a compound represented by the following general formula (c2-1),

R⁵-(CO)ₓO-(AO)_{y}-R⁶ (c2-1)

wherein R⁵ is an alkyl group or an alkenyl group with 9 or more and 18 or less carbons, R⁶ is a hydrogen atom or a methyl group, CO is a carbonyl group, x is a number of 0 or 1, AO is one or more alkyleneoxy groups selected from alkyleneoxy groups with 2 or more and 4 or less carbons, y is an average number of added moles and 3 or more and 50 or less, and when AO includes two or more alkyleneoxy groups, they may be bonded at random or bonded in blocks.

In the formula (c2-1), R⁵ has 9 or more, preferably 10 or more, more preferably 11 or more and further preferably 12 or more, and 17 or less, preferably 16 or less, more preferably 15 or less and further preferably 14 or less carbons from the viewpoint of high-temperature storage stability.

In the formula (c2-1), AO is one or more alkyleneoxy groups selected from alkyleneoxy groups with 2 or more and 4 or less carbons and preferably one or more alkyleneoxy groups selected from ethyleneoxy group and propyleneoxy group from the viewpoint of high-temperature storage stability.

In the formula (c2-1), x is a number of 0 or 1 and preferably 0 from the viewpoint of high-temperature storage stability.

In the formula (c2-1), y is 3 or more, preferably 5 or more, more preferably 7 or more, further preferably 8 or more, furthermore preferably 9 or more and furthermore preferably 10 or more, and 50 or less, preferably 40 or less, more preferably 30 or less, further preferably 20 or less and furthermore preferably 15 or less from the viewpoint of high-temperature storage stability.

In the formula (c2-1), R⁶ is preferably a hydrogen atom.

Examples of component (C2) include, for example, a compound represented by the general formula (c2-2) below from the viewpoint of high-temperature storage stability. This compound is a compound of the above general formula (c2-1) in which AO is ethyleneoxy group and propyleneoxy group.

R⁷-O-(EO)*ₛ*-(PO)ₜ-(EO)ᵣ-H (c2-2)

wherein in the formula, R⁷ is an alkyl group or an alkenyl group with 8 or more and 18 or less carbons, EO is ethyleneoxy group, PO is propyleneoxy group, and s, t and r are each an average number of added moles, where s is 0 or more and 30 or less, t is 0.1 or more and 5 or less, and r is 0 or more and 30 or less.

When the composition of the present invention contains component (C), the composition contains component (C) in an amount of preferably 1 mass% or more, more preferably 2 mass% or more, further preferably 5 mass% or more, furthermore preferably 10 mass% or more and furthermore preferably 20 mass% or more, and preferably 40 mass% or less, more preferably 35 mass% or less and further preferably 30 mass% or less from the viewpoints of dispersion stability of component (A) or other base agents in the composition, detergency when the composition is used as a detergent, and high-temperature storage stability.

In the composition of the present invention, a mass ratio of a content of component (C) to a content of component (A) in terms of the enclosed functional agents, (C)/(A), is preferably 0 or more, more preferably 5 or more, further preferably 10 or more, furthermore preferably 25 or more, furthermore preferably 50 or more and furthermore preferably 90 or more, and preferably 1000 or less, more preferably 700 or less, further preferably 500 or less and furthermore preferably 200 or less from the viewpoint of high-temperature storage stability.

The composition of the present invention can contain a thickener as component (D) to suppress separation of the silica capsule enclosing a functional agent of component (A). However, thickeners enclosed in component (A) are excluded from component (D). A content of component (D) in the composition of the present invention is preferably 0.05 mass% or more, more preferably 0.07 mass% or more and further preferably 0.1 mass% or more from the viewpoints of suppression of separation of component (A) and high-temperature storage stability, and preferably 1 mass% or less, more preferably 0.8 mass% or less and further preferably 0.5 mass% or less from the viewpoints of a reduced viscosity of the composition of the present invention and high-temperature storage stability.

As component (D), thickeners commonly used in compositions formulated with capsule particles can be used. From the viewpoints of suppression of separation of component (A) and high-temperature storage stability, for example, one or more selected from hydrogenated castor oil, acrylic acid-type polymers, polyethylene glycol, acrylamide-type polymers, cellulose nanofiber, polysaccharides and others can be used as component (D). Component (D) is preferably a thixotropic thickener from the viewpoint of changes in liquid viscosity during use.

The composition of the present invention can further contain an organic solvent having a hydroxy group as component (E) from the viewpoint of stable incorporation of component (C) and the viewpoint of high-temperature storage stability. However, organic solvents having a hydroxy group enclosed in component (A) are excluded from component (E).

Specific examples of component (E) can include the following compounds (E1) to (E6) from the viewpoint of high-temperature storage stability,
(E1) monohydric alcohols with 2 or more and 4 or less carbons such as ethanol, isopropanol and others,
(E2) dihydric or more and hexahydric or less polyhydric alcohols with 2 or more and 8 or less carbons such as ethylene glycol, propylene glycol, butylene glycol, hexylene glycol, glycerin and others,
(E3) glycol ethers with 4 or more and 12 or less carbons such as diethylene glycol, triethylene glycol, tetraethylene glycol, dipropylene glycol, tripropylene glycol and others,
(E4) alkyl (with 1 or more and 10 or less carbons) ethers of dihydric or more and tetrahydric or less polyhydric alcohols such as diethylene glycol monomethyl ether, diethylene glycol monoethyl ether, diethylene glycol monopropyl ether, diethylene glycol monobutyl ether, dipropylene glycol monomethyl ether, dipropylene glycol monoethyl ether, tripropylene glycol monomethyl ether, 1-methoxy-2-propanol, 1-ethoxy-2-propanol, 3-methyl-1,3-butanediol, 1-methylglyceryl ether, 2-methylglyceryl ether, 1,3-dimethylglyceryl ether, 1-ethylglyceryl ether, 1,3-diethylglyceryl ether, 1-pentylglyceryl ether, 2-pentylglyceryl ether, 1-octylglyceryl ether, 2-ethylhexylglyceryl ether, diethylene glycol monobutyl ether and others,
(E5) aromatic ethers of glycols such as phenoxyethanol, diethylene glycol monophenyl ether, triethylene glycol monophenyl ether, polyethylene glycol monophenyl ether with an average molecular weight of about 480, 2-benzyloxyethanol, diethylene glycol monobenzyl ether and others, and
(E6) organic solvents other than the above (E1) to (E5) such as 3-methoxy-3-methyl-1-butanol and others.

Component (E) is preferably one or more selected from diethylene glycol monobutyl ether, ethanol, ethylene glycol, propylene glycol and butylene glycol and more preferably one or more selected from diethylene glycol monobutyl ether, ethylene glycol and propylene glycol from the viewpoint of stable incorporation of component (C) and the viewpoint of high-temperature storage stability.

When the composition of the present invention contains component (E), the composition contains component (E) in an amount of preferably 1 mass% or more, more preferably 3 mass% or more and further preferably 5 mass% or more from the viewpoint of stable incorporation of component (C) and the viewpoint of high-temperature storage stability, and preferably 20 mass% or less, more preferably 18 mass% or less and further preferably 15 mass% or less from the viewpoint of suppression of leakage of the functional agents from the silica capsule in component (A) and the viewpoint of high-temperature storage stability.

The composition of the present invention can further contain a pH adjuster as component (F) from the viewpoints of suppression of precipitation or suppression of separation of solids in the composition in low-temperature environments and high-temperature storage stability. However, pH adjusters enclosed in component (A) are excluded from component (F).

Examples of the pH adjuster include a compound selected from
(1) acid agents such as inorganic acids such as hydrochloric acid, sulfuric acid and others, organic acids such as citric acid, succinic acid, malic acid, fumaric acid, tartaric acid, malonic acid, maleic acid and others, para-toluenesulfonic acid, xylene sulfonic acid and others, and
(2) alkali agents such as sodium hydroxide or potassium hydroxide, ammonia or derivatives thereof, amine salts such as monoethanolamine, diethanolamine, triethanolamine and others, or the like, sodium carbonate, potassium carbonate and others.

When the composition of the present invention contains component (F), the composition contains component (F) in an amount of preferably 0.01 mass% or more, more preferably 0.03 mass% or more and further preferably 0.05 mass% or more, and preferably 2 mass% or less, more preferably 1.5 mass% or less and further preferably 1.0 mass% or less from the viewpoints of suppression of precipitation or suppression of separation of solids in the composition in low-temperature environments and high-temperature storage stability.

The balance of the composition of the present invention is water. As the water, water commonly used in liquid detergents and others is used, and deionized water (ion exchange water) or water obtained by adding sodium hypochlorite in an amount of 1 mg/kg or more and 5 mg/kg or less to ion exchange water can be used. Further, distilled water or tap water can also be used.

The composition of the present invention contains water in an amount of preferably 50 mass% or more, more preferably 60 mass% or more and further preferably 65 mass% or more, and preferably 99.8 mass% or less, more preferably 95 mass% or less and further preferably 90 mass% or less in the composition from the viewpoint of high-temperature storage stability.

In addition to the above components, the composition of the present invention may be formulated with the following components (G1) to (G7) in the range that the effects of the present invention are not impaired. However, those which are enclosed in component (A) are excluded from these components.
(G1) recontamination prevention agents and dispersants such as polyacrylic acid, polymaleic acids, carboxymethyl cellulose and others
(G2) bleaching agents such as hydrogen peroxide, sodium percarbonate or sodium perborate and others
(G3) bleach activators such as tetraacetylethylenediamine, bleaching activators represented by the general formulas (I-2) to (I-7) of JP-A H6-316700 and others
(G4) one or more enzymes selected from cellulase, amylase, pectinase, protease and lipase
(G5) fluorescent dyes, for example, fluorescent dyes commercially available as Tinopal CBS (trade name, manufactured by Ciba Specialty Chemicals) or Whitex SA (trade name, manufactured by Sumitomo Chemical Co., Ltd.)
(G6) antioxidants such as butylhydroxytoluene, distyrenated cresol, sodium sulfite and sodium bisulfite and others
(G7) colorants, fragrances, antibacterial preservatives such as diclosan and others, and antifoaming agents such as a silicone and others

A pH of the composition of the present invention at 25°C is preferably 4 or more, more preferably 5 or more and further preferably 6 or more, and preferably 9 or less, more preferably 8.5 or less and further preferably 8 or less from the viewpoints of suppression of precipitation or suppression of separation of solids in the composition in low-temperature environments and high-temperature storage stability. The pH is measured in accordance with the pH measurement method described below.

### [Method of measuring pH]

A composite electrode for pH measurements (9615S measurement method model JF15 manufactured by HORIBA) is connected to a pH meter (pH/ion meter D-71 manufactured by HORIBA), and the power is turned on. As the pH electrode internal solution, a saturated potassium chloride aqueous solution (3.33 mol/L) is used. Next, a pH 4.01 standard solution (phthalate standard solution), a pH 6.86 standard solution (neutral phosphate standard solution) and a pH 9.18 standard solution (borate standard solution) are each filled into a 100-mL beaker and immersed in a constant temperature bath at 25°C for 30 minutes. A calibration operation of immersing the electrode for pH measurements for 3 minutes in a standard solution adjusted to a constant temperature is performed in the order of pH 6.86 -> pH 9.18 -> pH 4.01. A sample to be measured is adjusted to 25°C, the electrode of the pH meter is immersed in the sample, and a pH 3 minutes later is measured.

A viscosity of the composition of the present invention at 25°C is preferably 10 mPa·s or more, more preferably 20 mPa·s or more and further preferably 30 mPa·s or more, and preferably 400 mPa·s or less, more preferably 300 mPa·s or less and further preferably 200 mPa·s or less from the viewpoints of ease of handling and high-temperature storage stability. Note that these viscosities are measured using a B-type viscometer (manufactured by TOKYO KEIKI INC., VISCOMETER MODEL DVM-B) with rotor No. 3 or 4, a number of revolutions of 60 r/min and a measurement time of 60 seconds.

The composition of the present invention can be used for applications such as, for example, fiber treatment agents such as a detergent, a softener, an anti-creasing agent (for example, a spray-type anti-creasing agent) and others, additives for sanitary products such as a disposable diaper and others, fragrant cosmetics such as an aromatic, a milky lotion, a cosmetic liquid, a lotion, a serum, a cream, a gel preparation, a hair treatment agent, a quasi-drug and others, or the like.

In addition to the aforementioned embodiments, the present invention discloses the aspects below. The descriptions about the composition of the present invention can be modified as necessary and applied to these aspects. Further, the descriptions of each aspect can be modified as necessary and applied to another aspect.
<1> A composition containing the following components (A) and (B) and water,
component (A): a silica capsule enclosing a functional agent, and
component (B): a compound selected from a silicic acid and a silicate,
wherein component (B) is contained in the composition separately from component (A).

<2> The composition according to <1>, wherein component (A) has a shell containing silica as a constituent component and a core containing the functional agent inside the shell.
<3> The composition according to <2>, wherein the shell has a first shell and a second shell, and further has the first shell enveloping the core containing one or more functional agents and the second shell enveloping the first shell.
<4> The composition according to <3>, wherein the shell has a third shell composed of an organic polymeric compound in contact with the second shell.
<5> The composition according to any of <2> to <4>, wherein a thickness of the shell (which is a first shell when the shell has the first shell and a second shell) is preferably 5 nm or more, and preferably 20 nm or less and more preferably 15 nm or less.
<6> The composition according to any of <2> to <5>, wherein the shell (which is a first shell when the shell has the first shell and a second shell) is a dense layer having no pores.
<7> The composition according to any of <1> to <6>, wherein component (A) has a first shell and a second shell, and a thickness of the second shell is preferably 10 nm or more and more preferably 20 nm or more, and preferably 100 nm or less and more preferably 80 nm or less.
<8> The composition according to any of <1> to <7>, wherein component (A) has a first shell and a second shell, and the second shell is of a mesoporous structure, which is a higher order structure in which silica is present in a direction along an interface with the first shell and a thickness direction, where the "mesoporous structure" in the second shell is a structure that a diameter of pores present within the structure (so-called mesopores) falls within the range of preferably more than 2 nm, more preferably 10 nm or more and further preferably 30 nm or more, and preferably 50 nm or less, more preferably 45 nm or less and further preferably 40 nm or less.
<9> The composition according to any of <1> to <8>, wherein the functional agent of component (A) is one or more selected from a fragrance, a fragrance precursor, an oil agent, an antioxidant, a cooling sensation agent, a warming sensation agent, an antibacterial agent, a dye, a colorant, an ultraviolet absorber, a silicone, a solvent and an oil-soluble polymer, further one or more selected from a fragrance, a fragrance precursor, an oil agent, an antioxidant, a cooling sensation agent, a warming sensation agent, an antibacterial agent, an ultraviolet absorber and a solvent, and further one or more selected from a fragrance and a fragrance precursor.
<10> The composition according to any of <1> to <8>, wherein the functional agent of component (A) is one or more selected from a skincare component, a cosmetic oil, an antiseptic, an antioxidant, an insecticidal component and an insect-repellant component.
<11> The composition according to any of <1> to <10>, wherein a volume average particle size of component (A) is preferably 0.5 um or more, more preferably 0.7 um or more and further preferably 1 um or more, and preferably 50 um or less, more preferably 10 um or less and further preferably 5 um or less.
<12> The composition according to any of <1> to <11>, wherein component (A) has a first shell and a second shell containing silica as a constituent component and a core containing one or more functional agents inside the first shell, and component (A) is obtained by a production method including the following steps (1) and (2),
step (1): a step of forming a capsule enclosing the one or more functional agents, in which an organic phase containing the functional agents and a raw material silica (for example, a tetraalkoxysilane) is mixed and emulsified in an aqueous phase containing a surfactant (for example, a cationic surfactant), and a sol-gel reaction is thereafter carried out under an acidic condition to form a shell, and
step (2): a step of forming a capsule having the second shell enveloping the first shell, in which a raw material silica (for example, a tetraalkoxysilane) is further added to a dispersion liquid containing the capsule obtained in step (1), and a sol-gel reaction is carried out.

<13> The composition according to any of <1> to <12>, wherein component (A) has a first shell and a second shell containing silica as a constituent component and a core containing one or more functional agents inside the first shell, and component (A) is obtained by a production method including the following steps (1a) and (2a),
step (1a): a step of forming a capsule having the core and the first shell, in which an organic phase containing the one or more functional agents and a tetraalkoxysilane in an amount of 10 mass% or more and 60 mass% or less relative to the functional agents is emulsified in an aqueous phase containing a surfactant (for example, a cationic surfactant), and a sol-gel reaction is carried out under an acidic condition, and
step (2a): a step of forming a capsule having the second shell enveloping the first shell, in which a tetraalkoxysilane is further added to an aqueous dispersion containing the capsule obtained in step (1a), and a sol-gel reaction is carried out in such a manner that an initial pH of the sol-gel reaction of step (2a) is maintained lower than an initial pH of the sol-gel reaction of step (1a),
the production method further including the following step (3a) as necessary,
step (3a): a step of forming a capsule having a third shell, in which a dispersion liquid containing the capsule obtained in step (2a) is mixed with an aqueous solution of an organic polymeric compound (for example, an anionic synthetic polymeric compound).

<14> The composition according to <12> or <13>, wherein the sol-gel reactions in steps (1) and (2) and steps (1a) and (2a) are reactions of synthesizing silica of the first and second shells, in which a raw material silica (silica precursor) is polymerized with alcohol elimination through hydrolysis and polycondensation under an acidic condition.
<15> The composition according to any of <1> to <14>, wherein a content of the functional agent in component (A) is 5 mass% or more, further 10 mass% or more and further 12 mass% or more, and 50 mass% or less, further 45 mass% or less and further 40 mass% or less.
<16> The composition according to any of <1> to <15>, wherein a content of component (A) in terms of the enclosed functional agent is preferably 0.02 mass% or more, more preferably 0.05 mass% or more, further preferably 0.1 mass% or more and furthermore preferably 0.2 mass% or more, and preferably 10 mass% or less, more preferably 5 mass% or less and further preferably 1 mass% or less in the composition.
<17> The composition according to any of <1> to <16>, wherein component (B) is a compound represented by the following formula (B1),

M₂O·nSiO₂·mH₂O (B1)

wherein M is an atom selected from an alkali metal atom and a hydrogen atom, n is a number of 1.0 or more and 4.0 or less, and m is a number of 5.0 or more and 50.0 or less.
<18> The composition according to <17>, wherein in the formula (B1), the alkali metal atom of M is selected from a sodium atom and a potassium atom.
<19> The composition according to <17> or <18>, wherein in the formula (B1), n is a number of preferably 1.8 or more and 4.0 or less, more preferably from 1.9 to 3.5 and further preferably 2.0 or more and 3.3 or less.
<20> The composition according to any of <17> to <19>, wherein in the formula (B1), m is a number of preferably 10.0 or more and 48.0 or less, more preferably from 11.0 to 35.0 and further preferably 11.5 or more and 30.0 or less.
<21> The composition according to any of <1> to <20>, wherein component (B) is sodium silicate (sodium silicate), and further sodium silicate selected from sodium silicate No. 1, sodium silicate No. 2, sodium silicate No. 3, sodium silicate No. 4 and sodium silicate No. 5, or sodium silicate specified in JIS K 1408.
<22> The composition according to any of <1> to <21>, wherein component (B) is a compound selected from examples 1 to 9 in the following table:

| Example of silicate | | Composition (mass%) | | | | | Molar ratio | |
|---|---|---|---|---|---|---|---|---|
| | | Na₂O | SiO₂ | K₂O | H₂O | Total | n(SiO₂/M₂O) | m(H₂O/M₂O) |
| Sodium silicate | Example 1 | 15.6 | 31.8 | | 52.6 | 100.0 | 2.1 | 11.6 |
| | Example 2 | 11.8 | 28.6 | | 59.6 | 100.0 | 2.5 | 17.3 |
| | Example 3 | 9.5 | 29.1 | | 61.4 | 100.0 | 3.2 | 22.3 |
| | Example 4 | 7.7 | 25.0 | | 67.4 | 100.0 | 3.4 | 30.2 |
| | Example 5 | 7.1 | 25.7 | | 67.2 | 100.0 | 3.8 | 32.7 |
| Potassium silicate | Example 6 | | 28.0 | 22.0 | 50.0 | 100.0 | 2.0 | 11.9 |
| | Example 7 | | 21.0 | 9.0 | 70.0 | 100.0 | 3.7 | 40.7 |
| Sodium metasilicate | Example 8 | 28.9 | 27.5 | | 43.6 | 100.0 | 1.0 | 5.2 |
| | Example 9 | 22.0 | 21.0 | | 57.0 | 100.0 | 1.0 | 8.9 |

<23> The composition according to any of <1> to <22>, wherein the composition contains component (B) in an amount of preferably 0.001 mass% or more, more preferably 0.01 mass% or more, further preferably 0.05 mass% or more and furthermore preferably 0.15 mass% or more, and preferably 0.5 mass% or less, more preferably 0.4 mass% or less and further preferably 0.3 mass% or less.
<24> The composition according to any of <1> to <23>, wherein a mass ratio of a content of component (B) in terms of silicon dioxide (SiO₂) to a content of component (A) in terms of the enclosed functional agent, (B)/(A), is preferably 0.01 or more, more preferably 0.05 or more, further preferably 0.25 or more and furthermore preferably 0.75 or more, and preferably 1.0 or less.
<25> The composition according to any of <1> to <24>, further containing the following component (C),
component (C): a surfactant.
<26> The composition according to <25>, wherein component (C) is one or more surfactants selected from (C1) an anionic surfactant (hereinafter referred to as component (C1)) and (C2) a nonionic surfactant, and further one or more surfactants selected from (C2) a nonionic surfactant (hereinafter referred to as component (C2)).
<27> The composition according to <26>, wherein the anionic surfactant of component (C1) is an anionic surfactant selected from a sulfonic acid having a hydrocarbon group and a salt thereof, a sulfate having a hydrocarbon group and a salt thereof, and a carboxylic acid having a hydrocarbon group and a salt thereof, and further an anionic surfactant selected from a sulfonate having a hydrocarbon group and a carboxylate having a hydrocarbon group.
<28> The composition according to <27>, wherein the hydrocarbon groups are alkyl groups or alkenyl groups.
<29> The composition according to <27> or <28>, wherein the hydrocarbon groups have 3 or more, preferably 7 or more, more preferably 9 or more and further preferably 11 or more, and preferably 22 or less, more preferably 20 or less and further preferably 18 or less carbons.
<30> The composition according to any of <27> to <29>, wherein the salts of the anionic surfactants are salts selected from monovalent metal salts such as sodium salts, potassium salts and others, divalent metal salts such as magnesium salts and others, ammonium salts, and organic amine salts such as monoethanolamine salts, diethanolamine salts, triethanolamine salts and others, and further sodium salts.
<31> The composition according to any of <26> to <30>, wherein component (C1) is one or more anionic surfactants selected from the following components (c1-1) to (c1-5), and further one or more anionic surfactants selected from the following component (c1-1),
component (c1-1): a sulfonic acid or a salt thereof represented by the following general formula (c1-1),

   R¹-B-SO₃M (c1-1)
wherein in the formula (c1-1), R¹ represents an alkyl group or an alkenyl group with 3 or more and 21 or less carbons, B represents a benzene ring, M represents a hydrogen atom, an alkali metal, an alkaline earth metal (1/2 atom), ammonium or an organic ammonium, and the sulfonic acid group is bonded in an ortho, meta or para position relative to R¹ bonded to B,
component (c1-2): a salt of an internal olefin sulfonic acid with 14 or more and 24 or less carbons,
component (c1-3): a salt of a fatty acid with 8 or more and 20 or less carbons,
component (c1-4): a sulfate or a salt thereof represented by the following general formula (c1-4),

   R²-O-[(PO)ₘ/(EO)ₙ]-SO₃M (c1-4)
wherein in the formula (c1-4), R² represents an alkyl group or an alkenyl group with 8 or more and 22 or less carbons, in which a carbon atom bonded to the oxygen atom is a primary carbon atom, PO represents propyleneoxy group, EO represents ethyleneoxy group, EO and PO may be bonded in blocks or at random, / is a sign indicating that PO and EO are bonded in any order, m and n are average numbers of added moles, where m is 0 or more and 5 or less and n is 0 or more and 16 or less, and M represents a hydrogen atom, an alkali metal, an alkaline earth metal (1/2 atom), ammonium or an organic ammonium, and
component (c1-5): an α-sulfo fatty acid ester or a salt thereof represented by the following general formula (c1-5),

   R³-CH(SO₃M)COOR⁴ (c1-5)
wherein in the formula (c1-5), R³ represents an alkyl group or an alkenyl group with 6 or more and 20 or less carbons, R⁴ represents an alkyl group with 1 or more and 6 or less carbons, and M represents a hydrogen atom, an alkali metal, an alkaline earth metal (1/2 atom), ammonium or an organic ammonium.

<32> The composition according to <31>, wherein in the formula (c1-1), R¹ has 3 or more, preferably 5 or more, more preferably 6 or more and further preferably 7 or more, and 21 or less, preferably 20 or less, more preferably 19 or less and further preferably 18 or less carbons.
<33> The composition according to <31> or <32>, wherein in the formula (c1-1), M is an alkali metal or an organic ammonium, and further sodium.
<34> The composition according to any of <31> to <33>, wherein component (c1-1) is a surfactant selected from an alkyl (with 4 or more carbons) benzene sulfonic acid and a cumene sulfonic acid.
<35> The composition according to any of <31> to <34>, wherein the internal olefin sulfonate of component (c1-2) has 14 or more, preferably 16 or more and more preferably 18 or more, and 24 or less, more preferably 22 or less and further preferably 20 or less carbons.
<36> The composition according to any of <31> to <35>, wherein the salt of component (c1-2) is a salt selected from an alkali metal salt such as sodium, potassium or the like, an alkaline earth metal salt such as calcium, magnesium or the like, an ammonium salt, and an organic ammonium salt, for example, an alkanol ammonium salt such as monoethanolammonium, diethanolammonium, triethanolammonium or the like, and further a salt selected from an alkali metal salt, and an alkanol ammonium salt with 2 or more and 6 or less carbons.
<37> The composition according to any of <31> to <33>, wherein the fatty acid of component (c1-3) has 8 or more, preferably 10 or more and more preferably 12 or more, and 20 or less, more preferably 18 or less and further preferably 16 or less carbons.
<38> The composition according to any of <31> to <37>, wherein the salt of component (c1-3) is an alkali metal salt, an alkaline earth metal (1/2 atom) salt, an ammonium salt or an organic ammonium salt.
<39> The composition according to any of <31> to <38>, wherein component (c1-3) is a salt of a fatty acid selected from an octanoic acid salt, a decanoic acid salt, a lauric acid salt, a myristic acid salt, a palmitic acid salt, a stearic acid salt, a coconut fatty acid salt, a palm fatty acid salt and a palm kernel fatty acid salt.
<40> The composition according to any of <31> to <39>, wherein in the formula (c1-4), R² is an alkyl group or an alkenyl group, further an alkyl group and further a linear alkyl group with 8 or more, preferably 9 or more, more preferably 10 or more and further preferably 12 or more, and 22 or less, preferably 18 or less, more preferably 16 or less and further preferably 14 or less carbons.
<41> The composition according to any of <31> to <40>, wherein in the formula (c1-4), m is preferably 4 or less and more preferably 3 or less.
<42> The composition according to any of <31> to <41>, wherein in the formula (c1-4), n is 0 or more, preferably 1 or more, more preferably 2 or more and further preferably 4 or more, and preferably 10 or less, more preferably 8 or less and further preferably 6 or less.
<43> The composition according to any of <31> to <42>, wherein in the formula (c1-4), M is preferably a hydrogen atom, an alkali metal such as sodium, potassium or the like, an alkaline earth metal (1/2 atom) such as magnesium, calcium or the like, or an organic ammonium, more preferably an alkali metal such as sodium, potassium or the like, or an alkanol ammonium such as monoethanolammonium, diethanolammonium or the like, and further preferably sodium.
<44> The composition according to any of <31> to <43>, wherein component (c1-4) is a (polyoxypropylene) polyoxyethylene alkyl ether sulfate sodium salt in which the alkyl group has 12 or more and 14 or less carbons, an average number of added moles of propyleneoxy group is 0 or more and 4 or less, and an average number of added moles of ethyleneoxy group is 1 or more and 4 or less, in other words, component (c1-4) is a compound of the general formula (c1-4) in which R² is an alkyl group with 12 or more and 14 or less carbons, m is 0 or more and 4 or less, n is 1 or more and 4 or less, and M is sodium.
<45> The composition according to any of <31> to <44>, wherein in the formula (c1-5), R³ is an alkyl group or an alkenyl group and further an alkyl group with 6 or more, preferably 8 or more and more preferably 10 or more, and 20 or less, preferably 18 or less and more preferably 16 or less carbons.
<46> The composition according to any of <31> to <45>, wherein in the formula (c1-5), R⁴ is an alkyl group with 1 or more, and preferably 5 or less and more preferably 4 or less carbons.
<47> The composition according to any of <31> to <46>, wherein in the formula (c1-5), M is preferably a hydrogen atom, an alkali metal such as sodium, potassium or the like, an alkaline earth metal (1/2 atom) such as magnesium, calcium or the like, or an organic ammonium, more preferably an alkali metal such as sodium, potassium or the like, or an alkanol ammonium such as monoethanolammonium, diethanolammonium or the like, and further preferably sodium.
<48> The composition according to any of <31> to <47>, wherein component (c1-5) is a sodium α-sulfo fatty acid methyl ester or a salt thereof of the formula (c1-5) in which R³ is an alkyl group with 11 or more and 14 or less carbons and R⁴ is a methyl group.
<49> The composition according to any of <26> to <48>, wherein the nonionic surfactant of component (C2) is a nonionic surfactant selected from a sucrose fatty acid ester, a glycerin fatty acid ester, a sorbitan fatty acid ester, a polyoxyalkylene sorbitan fatty acid ester, a polyoxyalkylene fatty acid ester, a fatty acid alkanolamide or an alkylene oxide adduct thereof, a polyoxyalkylene alkyl ether, an alkyl glycoside, a polyoxyalkylene alkyl ether and a glyceryl monoether, and further a nonionic surfactant selected from a polyoxyalkylene ether and a fatty acid methyl ester alkoxylate.
<50> The composition according to any of <26> to <49>, wherein component (C2) is a compound represented by the following general formula (c2-1),

R⁵-(CO)ₓO-(AO)_{y}-R⁶ (c2-1)

wherein R⁵ is an alkyl group or an alkenyl group with 9 or more and 18 or less carbons, R⁶ is a hydrogen atom or a methyl group, CO is a carbonyl group, x is a number of 0 or 1, AO is one or more alkyleneoxy groups selected from alkyleneoxy groups with 2 or more and 4 or less carbons, y is an average number of added moles and 3 or more and 50 or less, and when AO includes two or more alkyleneoxy groups, they may be bonded at random or bonded in blocks.
<51> The composition according to <50>, wherein in the formula (c2-1), R⁵ has 9 or more, preferably 10 or more, more preferably 11 or more and further preferably 12 or more, and 17 or less, preferably 16 or less, more preferably 15 or less and further preferably 14 or less carbons.
<52> The composition according to <50> or <51>, wherein in the formula (c2-1), AO is one or more alkyleneoxy groups selected from ethyleneoxy group and propyleneoxy group.
<53> The composition according to any of <50> to <52>, wherein in the formula (c2-1), x is 0.
<54> The composition according to any of <50> to <53>, wherein in the formula (c2-1), y is 3 or more, preferably 5 or more, more preferably 7 or more, further preferably 8 or more, furthermore preferably 9 or more and furthermore preferably 10 or more, and 50 or less, preferably 40 or less, more preferably 30 or less, further preferably 20 or less and furthermore preferably 15 or less.
<55> The composition according to any of <50> to <54>, wherein in the formula (c2-1), R⁶ is a hydrogen atom.
<56> The composition according to any of <26> to <49>, wherein component (C2) is a compound represented by the following general formula (c2-2),

R⁷-O-(EO)ₛ-(PO)ₜ-(EO)ᵣ-H (c2-2)

wherein in the formula, R⁷ is an alkyl group or an alkenyl group with 8 or more and 18 or less carbons, EO is ethyleneoxy group, PO is propyleneoxy group, and s, t and r are each an average number of added moles, where s is 0 or more and 30 or less, t is 0.1 or more and 5 or less, and r is 0 or more and 30 or less.
<57> The composition according to any of <25> to <56>, wherein the composition contains component (C) in an amount of preferably 1 mass% or more, more preferably 2 mass% or more, further preferably 5 mass% or more, furthermore preferably 10 mass% or more and furthermore preferably 20 mass% or more, and preferably 40 mass% or less, more preferably 35 mass% or less and further preferably 30 mass% or less.
<58> The composition according to any of <25> to <57>, wherein a mass ratio of a content of component (C) to a content of component (A) in terms of the enclosed functional agent, (C)/(A), is preferably 0 or more, more preferably 5 or more, further preferably 10 or more, furthermore preferably 25 or more, furthermore preferably 50 or more and furthermore preferably 90 or more, and preferably 1000 or less, more preferably 700 or less, further preferably 500 or less and furthermore preferably 200 or less.
<59> The composition according to any of <1> to <58>, further containing the following component (D),
component (D): a thickener.
<60> The composition according to <59>, wherein the composition contains component (D) in an amount of 0.05 mass% or more, further 0.07 mass% or more and further 0.1 mass% or more, and 1 mass% or less, further 0.8 mass% or less and further 0.5 mass% or less.
<61> Use of the composition according to any of <1> to <60> as a fiber treatment agent, an additive for sanitary products or a fragrant cosmetic.
<62> The use according to <61>, wherein the fiber treatment agent is a detergent, a softener or an anti-creasing agent.
<63> The use according to <61> or <62>, wherein the additive for sanitary products is an additive for disposable diapers.
<64> The use according to any of <61> to <63>, wherein the fragrant cosmetic is one or more selected from an aromatic, a milky lotion, a cosmetic liquid, a lotion, a serum, a cream, a gel preparation, a hair treatment agent and a quasi-drug.
<65> An application of the composition according to any of <1> to <60> for use as a fiber treatment agent, an additive for sanitary products or a fragrant cosmetic.
<66> The application according to <65>, wherein the fiber treatment agent is one or more selected from a detergent, a softener and an anti-creasing agent.
<67> The application according to <65> or <66>, wherein the additive for sanitary products is an additive for disposable diapers.
<68> The application according to any of <65> to <67>, wherein the fragrant cosmetic is one or more selected from an aromatic, a milky lotion, a cosmetic liquid, a lotion, a serum, a cream, a gel preparation, a hair treatment agent and a quasi-drug.

### Examples

### <Example and comparative example>

The compositions shown in Tables 2 to 6 were prepared using the formulation components below, and evaluations of storage stability and others were made by the methods described later using the obtained compositions.

### <Formulation component>

### [Component (A)]

· A-1: a silica capsule enclosing a fragrance obtained by the following method 1

### [Method 1]

### Step (1)

3.0 g of QUARTAMIN 60W (trade name, manufactured by Kao Corporation, cetyltrimethylammonium chloride, an effective component amount of 30 mass%) was diluted with 750 g of ion exchange water to obtain an aqueous phase component. An oil phase component prepared by mixing 200 g of model fragrance A1 of the formulation proportions shown in Table 1 and 50 g of tetraethoxysilane (hereinafter also referred to as "TEOS") was added to this aqueous phase component, and the mixed liquid was made to be emulsified using a homo mixer (manufactured by HsiangTai, model: HM-310, the same applies hereinafter) at a number of revolutions of 8,500 rpm, thus obtaining an emulsion. A volume average particle size of emulsion droplets was then 1.4 um.

After adjusted to have a pH of 3.8 using a 1% aqueous sulfuric acid solution, the obtained emulsion was transferred to a separable flask provided with a stirring blade and a cooler, and stirred at 200 rpm for 24 hours while kept at a liquid temperature of 30°C, thus obtaining an aqueous dispersion containing silica capsules having a core composed of model fragrance A1 and a first shell composed of silica.

### Step (2)

While the aqueous dispersion obtained in step 1 was stirred at a liquid temperature of 30°C, 21 g of TEOS was added dropwise thereto for 420 minutes. After dropwise addition, the aqueous dispersion was continuously further stirred for 17 hours and then cooled to form a second shell enveloping the first shell, thus obtaining an aqueous dispersion containing silica capsules in which model fragrance A1 was enclosed in amorphous silica (a content of model fragrance A1 (functional agent) in the silica capsules was 19.4 mass%). A volume average particle size of silica capsules was 2.1 um. The volume average particle sizes of emulsion droplets and silica capsules (I) were measured using the laser diffraction/scattering particle size distribution measurement device "LA-960" (trade name, manufactured by HORIBA, Ltd.) For measurements, a flow cell was used, the medium was water, and the refractive index was set at 1.40-0i. The emulsion or the aqueous dispersion containing silica capsules was added to the flow cell, measurements were made at a concentration at which a transmittance near 90% was indicated, and a volume average particle size was determined on a volume basis (the same applies to a measurement method of a volume average particle size of another type of component (A)).

Note that a thickness of the first shell was about 5 nm and a thickness of the second shell was 5 to 30 nm.

Note that, as model fragrance A1, a fragrance having the composition shown in Table 1 below (volume average ClogP: 3.9, specific gravity: 0.96) was used. Note that the above volume average ClogP value of model fragrance A1 was calculated as a sum of ClogP values of fragrance components contained in model fragrance A1 multiplied by their respective volume fractions in model fragrance A1. In this calculation, all fragrance components whose contents in model fragrance A1 were 0.5 mass% or more were considered, and fragrance components whose contents in model fragrance A1 were less than 0.5 mass% and whose specific gravity and ClogP values were known were also included in the calculation.

**[Table 1]**

| Model fragrance A1 | | |
|---|---|---|
| Fragrance component | | |
| Type | Content (mass%) | ClogP |
| Methyl dihydrojasmonate | 19.7 | 3.0 |
| γ-decalactone | 13.0 | 2.6 |
| Ethylene brassylate | 11.5 | 4.7 |
| o,t-butylcyclohexyl acetate | 10.6 | 4.4 |
| AMBER CORE | 7.7 | 4.1 |
| Others | 37.5 | - |

· A-2: a silica capsule enclosing a fragrance obtained by the following method 2

### [Method 2]

1.80 g of the trade name "QUARTAMIN 60W" (manufactured by Kao Corporation; cetyltrimethylammonium chloride, an effective component amount of 30 mass%) was diluted with 448.20 g of ion exchange water to prepare an aqueous solution. An oil phase prepared by mixing 120 g of model fragrance A2 and 30 g of tetraethoxysilane (TEOS) was added to this aqueous solution, and the mixed liquid was made to be emulsified using the homo mixer set at a number of revolutions of 8,500 rpm. After adjusted to have a pH of 3.7 using 1 wt% of sulfuric acid, the emulsion was transferred to a separable flask provided with a stirring blade and a cooler, and stirred at 160 rpm for 17 hours while kept at a liquid temperature of 30°C, thus obtaining a suspension containing silica capsules with a volume average particle size of 1.9 um in which model fragrance A2 was encapsulated in amorphous silica (a content of model fragrance A2 (functional agent) in the silica capsules was 20 mass%).

Note that the composition of model fragrance A2 was 25 mass% of limonene, 25 mass% of methyl-iso-eugenol and 50 mass% of α-hexylcinnamaldehyde.

· A-3: a silica capsule enclosing a cooling sensation imparting agent obtained by the following method 3

### [Method 3]

### Step (1)

0.90 g of the trade name "QUARTAMIN 60W" (manufactured by Kao Corporation; cetyltrimethylammonium chloride, an effective component amount of 30 mass%) was diluted with 224.10 g of ion exchange water to prepare an aqueous solution. An oil phase prepared by mixing 60 g of a cooling sensation imparting agent and 15 g of tetraethoxysilane (TEOS) was added to this aqueous solution, and the mixed liquid was made to be emulsified using the homo mixer set at a number of revolutions of 8,500 rpm. After adjusted to have a pH of 3.7 using 1 wt% of sulfuric acid, the emulsion was transferred to a separable flask provided with a stirring blade and a cooler, and stirred at 160 rpm for 24 hours while kept at a liquid temperature of 30°C.

### Step (2)

Next, while the liquid temperature was kept at 30°C, dropwise addition of 9.0 g of TEOS was carried out for 420 minutes at a suspension pH of 3.7. After dropwise addition, the suspension was continuously further stirred for 24 hours and then cooled, thus obtaining a suspension containing silica capsules with a volume average particle size of 1.4 um in which the cooling sensation imparting agent was encapsulated in amorphous silica (a content of the cooling sensation imparting agent (functional agent) in the silica capsules was 20 mass%).

Note that the composition of the cooling sensation imparting agent was 60 mass% of l-menthol, 10 mass% of isopropyl myristate and 30 mass% of hexyl salicylate.

· A-4: a silica capsule enclosing an ultraviolet absorber (a content of the ultraviolet absorber (functional agent) in the silica capsule is 37 mass%), Eusolex UV-Pearls OB-S (product name) by Merck KGaA

### [Component (B)]

· B-1: sodium silicate (sodium silicate No. 2, FUJI CHEMICAL CO., LTD., Na₂O/SiO₂/H₂O = 11.8/28.6/59.6 (mass%))
· B-2: sodium silicate (sodium silicate No. 1, FUJI CHEMICAL CO., LTD., Na₂O/SiO₂/H₂O = 15.6/31.8/52.6 (mass%))
· B-3: sodium silicate (sodium silicate No. 3, FUJI CHEMICAL CO., LTD., Na₂O/SiO₂/H₂O = 9.5/29.1/61.4 (mass%)
· B-4: sodium silicate (sodium silicate No. 4, FUJI CHEMICAL CO., LTD., Na₂O/SiO₂/H₂O = 7.7/25.0/67.4 (mass%)
· B-5: sodium silicate (sodium silicate No. 5, FUJI CHEMICAL CO., LTD., Na₂O/SiO₂/H₂O = 7.1/25.7/67.2 (mass%)

### [Component (C)]

· C-1: sodium polyoxyethylene dodecyl ether sulfate (in which an average number of added moles of ethylene oxide (hereinafter EO) was 3)
· C-2: polyoxyethylene lauryl ether (in which an average number of added moles of EO was 10 moles)
· C-3: a potassium salt of an internal olefin sulfonic acid with 18 carbons. In C-3, a mass ratio of olefin species (potassium olefin sulfonates)/hydroxy species (potassium hydroxy alkane sulfonates) was 16/84. A mass ratio in a position distribution of a sulfonic acid group in hydroxy species in C-3 was the following: position 1/position 2/position 3/position 4/position 5/positions 6 to 9 = 1.5/22.1/17.2/21.8/13.5/23.9. Further, (IO-1S)/(IO-2S) was equal to 1.6 (mass ratio). Here, (IO-1S)/(IO-2S) is a mass ratio of a content of internal olefin sulfonate (IO-1S) in which a sulfonic acid group is present in position 2 or more and position 4 or less to a content of internal olefin sulfonate (IO-2S) in which a sulfonic acid group is present in position 5 or more in C-3.

Note that the position distribution of a sulfonic acid group of hydroxy species contained in C-3 was measured by liquid chromatography-mass spectrometry (hereinafter abbreviated as LC-MS). Note that an internal olefin sulfonate in which a double bond is present in position 6 or more could not be clearly fractionated due to overlapping peaks. The devices and analysis conditions used for the measurements are the following.

### [Measurement instrument]

LC device: "LC-20ASXR" (manufactured by SHIMADZU CORPORATION)
LC-MS device: "LCMS-2020" (manufactured by SHIMADZU CORPORATION)
Column: ODS Hypersil (length: 250 mm, inner diameter: 4.6 mm, particle size: 3 um, manufactured by Thermo Fisher Scientific Inc.)
Detector: ESI (-), m/z = 349.15 (C18), 321.10 (C16) and 293.05 (C14)

### [Solvent]

Solvent A: 10 mM ammonium acetate aqueous solution
Solvent B: acetonitrile/water = 95/5 solution with 10 mM ammonium acetate added

### [Elution conditions]

Gradient: solvent A 60%, solvent B 40% (0-15 min) -> solvent A 30%, solvent B 70% (15.1-20 min) -> solvent A 60%, solvent B 40% (20.1-30 min)
Flow rate: 0.5 ml/min
Column temperature: 40°C
Injection volume: 5 ul

· C-4: sodium alkylbenzene sulfonate (alkyl composition: C10/C11/C12/C13 = 11/29/34/26 (mass ratio), mass average carbon number = 17.75)
· C-5: polyoxyalkylene lauryl ether (a compound obtained by adding an average of 9 moles of EO, then adding an average of 2 moles of propylene oxide (hereinafter PO) and further adding an average of 9 moles of EO to 1 mole of lauryl alcohol)
· C-6: polyoxyalkylene lauryl ether (a compound obtained by adding an average of 3.7 moles of PO and then adding an average of 16.5 moles of EO to 1 mole of lauryl alcohol)
· C-7: polyoxyethylene lauryl ether (in which an average number of added moles of EO was 8.3 moles)
· C-8: polyoxyethylene lauryl ether (in which an average number of added moles of EO was 13.2 moles)
· C-9: polyoxyethylene lauryl ether (in which an average number of added moles of EO was 18.5 moles)

### [Other optional components]

· Propylene glycol: reagent, FUJIFILM Wako Pure Chemical Corporation
· Diethylene glycol monobutyl ether: reagent, FUJIFILM Wako Pure Chemical Corporation
· Hydrogenated castor oil: iodine value 1.5 g-I2/100 g
· Anti-foaming agent: DOWSIL AC8066 Antifoam, manufactured by Dow Corning Toray Co., Ltd.
· pH adjuster: citric acid, monoethanolamine

### <Method for preparing composition>

First, components other than components (A) and (B) of the formulation components shown in the tables were mixed at predetermined proportions. Subsequently, component (B) was mixed, and then, component (A) was mixed to prepare the compositions in the tables. At that time, for A-1 to A-3, the aqueous dispersion or the suspensions obtained by methods 1 to 3 were used, and for A-4, the product was used as-is, in such a manner that their formulation amounts in terms of the functional agents enclosed in component (A) (silica capsules) were the mass percentages shown in the tables. In other words, the mass percentages or the mass ratios for component (A) in the tables are based on the amounts in terms of the functional agents. Note that each composition was appropriately adjusted with a pH adjuster such that its pH (25C°) was the value shown in the tables. In the tables, "+" of a formulation amount of a pH adjuster means that the pH adjuster was used in an amount to make a pH of a composition the predetermined value.

### <Evaluation of storage stability at high temperature>

10 g of each composition in the tables was placed in a plastic container (15-mL laboratory centrifuge, manufactured by IWAKI) and sealed with a cap, and then, placed in a constant temperature bath at 50°C and stored for one week. L* was measured for appearances before and after storage using a colorimeter (manufactured by NIPPON DENSHOKU INDUSTRIES Co., Ltd., Spectrophotometer SE600) and upon performing calibration with a reflection mode and a standard plate No. 2. 5 mL of each composition before storage or after storage was added to a circular cell (inner diameter 3.5 cm, depth 1.5 cm) with a dropper so as not to foam, and used as a sample to measure L*.

### <Evaluation of appearance change>

A ΔL* value was calculated in accordance with the formula below using the L* values before storage and after storage at 50°C for one week measured by the above method. ΔL* = L* value after storage at 50°C for one week - L* value before storage

A ΔL* value closer to 0 indicates that there was less change in appearance, which is preferable.

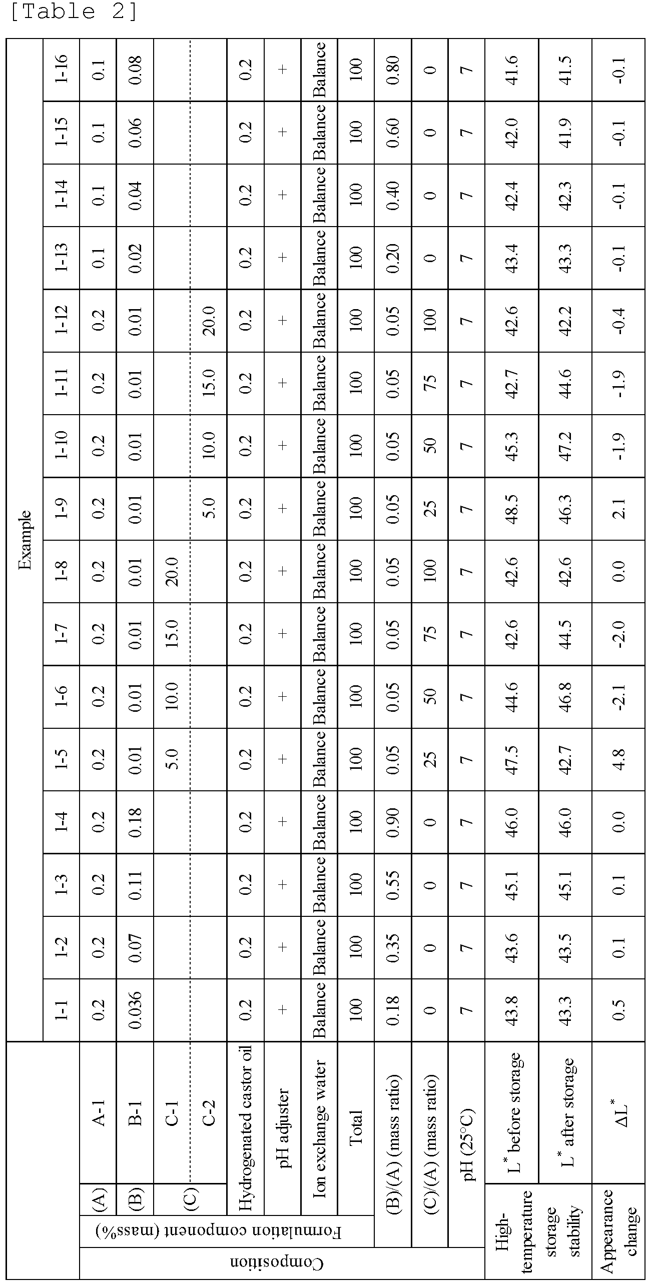

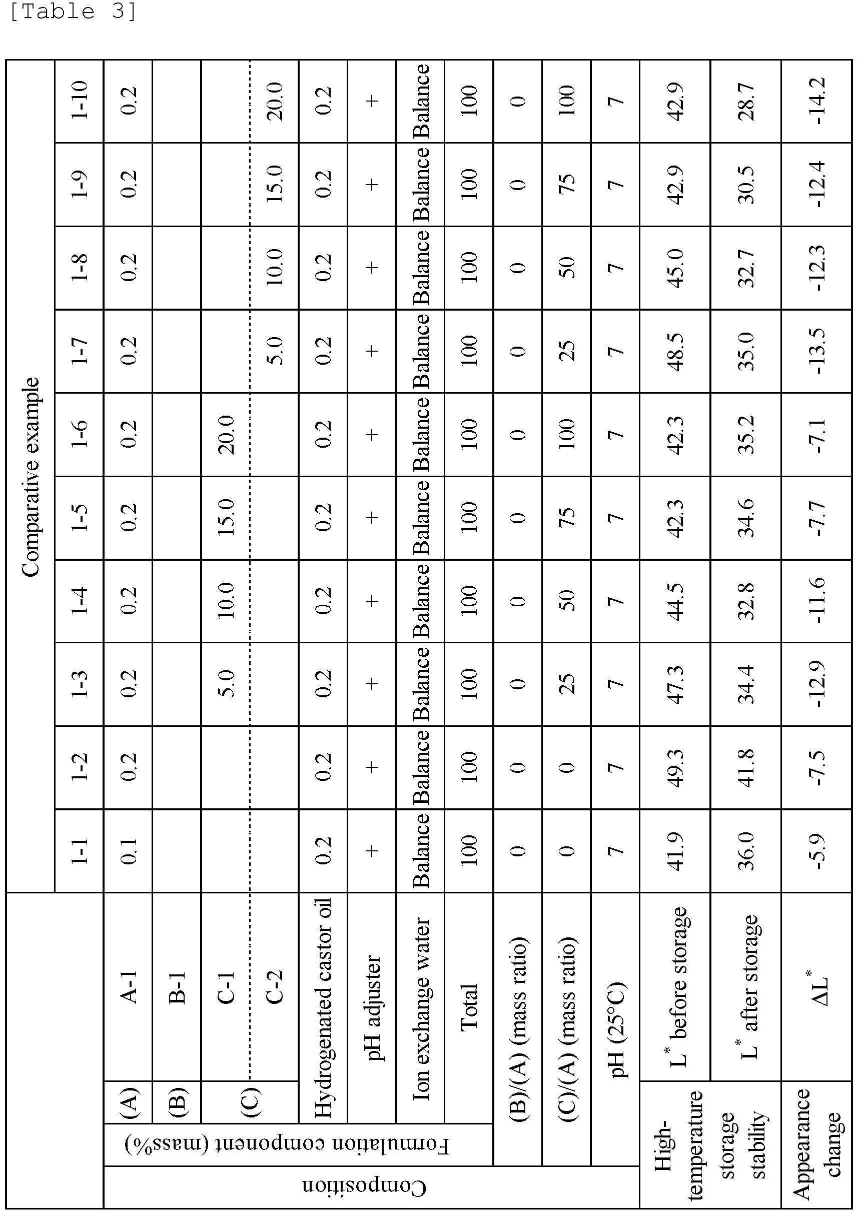

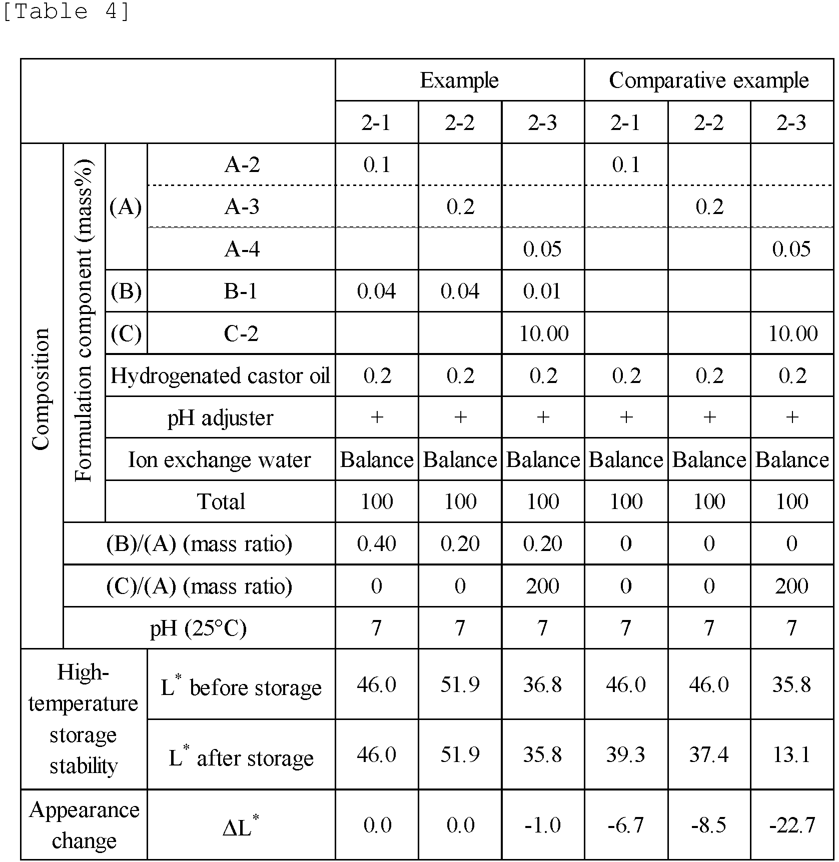

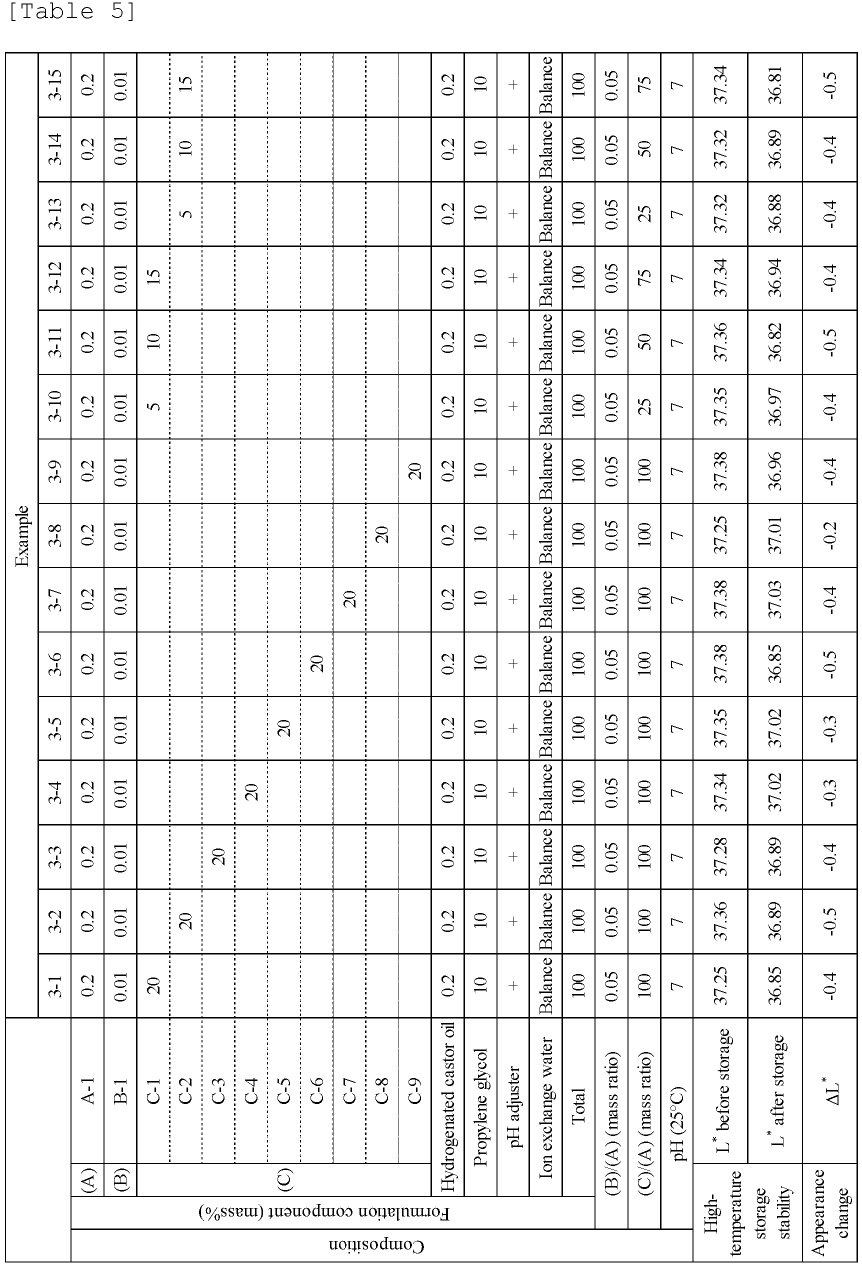

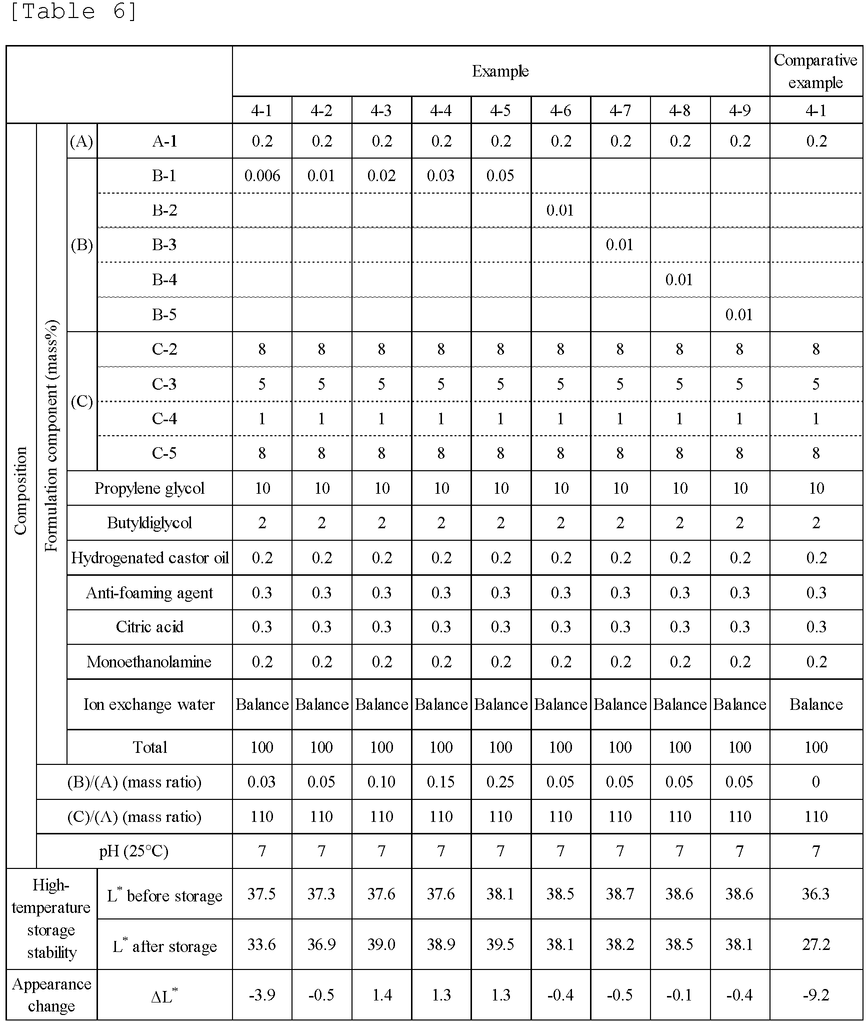

The contents of component (B) in the tables are contents in terms of silicon dioxide (SiO₂), and were quantified by the method below.

### <Quantification of silicon dioxide (SiO₂) in composition>

Each composition was filtered with a 0.2 um membrane filter. After 5 g of each sample was precisely weighed in a platinum crucible and subjected to an ashing process, the resulting product was melted in an electric furnace with 1 g of an alkali flux (sodium carbonate/boric acid = 1:0.4, mass ratio) added, then dissolved in added ultrapure water and 5 mL of 6N hydrochloric acid and cooled, and thereafter made to be a fixed volume in a 50-mL PP volumetric flask. This sample was subjected to ICP emission spectrometry using iCAP6500Duo manufactured by Thermo Fisher Scientific Inc., and a salt dissolved in the sample was calculated in terms of SiO₂. A calibration curve was prepared using a sample obtained by diluting a silicon standard solution (1000 ppm) and adding the same amount of the alkali flux.

## Claims

1. A composition comprising the following components (A) and (B) and water,
component (A): a silica capsule enclosing a functional agent, and
component (B): a compound selected from a silicic acid and a silicate,
wherein the component (B) is contained in the composition separately from the component (A).

2. The composition according to claim 1, wherein the component (B) is a compound represented by the following formula (B1),
M₂O·nSiO₂·mH₂O (B1)
wherein M is an atom selected from an alkali metal atom and a hydrogen atom, n is a number of 1.0 or more and 4.0 or less, and m is a number of 5.0 or more and 50.0 or less.

3. The composition according to claim 1 or 2, wherein a mass ratio of a content of the component (B) in terms of silicon dioxide to a content of the component (A) in terms of the enclosed functional agent, (B)/(A), is 0.05 or more.

4. The composition according to any one of claims 1 to 3, further comprising the following component (C),
component (C): a surfactant.

5. The composition according to claim 4, wherein a mass ratio of a content of the component (C) to a content of the component (A) in terms of the enclosed functional agent, (C)/(A), is 90 or more.

6. The composition according to any one of claims 1 to 5, wherein the functional agent of the component (A) is one or more selected from a fragrance, a fragrance precursor, an oil agent, an antioxidant, a cooling sensation agent, a warming sensation agent, an antibacterial agent, a dye, a colorant, an ultraviolet absorber, a silicone, a solvent and an oil-soluble polymer.

7. Use of the composition according to any one of claims 1 to 6 as a fiber treatment agent, an additive for sanitary products or a fragrant cosmetic.

8. An application of the composition according to any one of claims 1 to 6 for use as a fiber treatment agent, an additive for sanitary products or a fragrant cosmetic.
